(19) Europäisches Patentamt / European Patent Office / Office européen des brevets

(11) **EP 1 839 667 B1**

(12) **EUROPEAN PATENT SPECIFICATION**

(45) Date of publication and mention
of the grant of the patent:
**17.06.2015 Bulletin 2015/25**

(51) Int Cl.:
*A61K 35/36* (2015.01)     *A61K 48/00* (2006.01)
*A61K 38/18* (2006.01)     *A61P 11/00* (2006.01)
*C07K 14/52* (2006.01)     *A61K 38/44* (2006.01)

(21) Application number: **07009031.1**

(22) Date of filing: **23.09.1999**

(54) **Fibroblast and endothelial cell-based gene therapy for the pulmonary system**

Fibroblast- und Endothelialzellbasierte Gentherapie für das Lungensystem

Thérapie de gène à base de cellules de fibroblastes et endothéliales pour le système pulmonaire

(84) Designated Contracting States:
**AT BE CH CY DE DK ES FI FR GB GR IE IT LI LU
MC NL PT SE**

(43) Date of publication of application:
**03.10.2007 Bulletin 2007/40**

(62) Document number(s) of the earlier application(s) in
accordance with Art. 76 EPC:
**99944205.6 / 1 220 679**

(73) Proprietor: **Northern Therapeutics Inc.
Ottawa, ON K1H 8L6 (CA)**

(72) Inventor: **Stewart, Duncan J.
Toronto
Ontario M4P 2K2 (CA)**

(74) Representative: **Vossius & Partner
Patentanwälte Rechtsanwälte mbB
Siebertstrasse 3
81675 München (DE)**

(56) References cited:
**EP-A2- 0 893 493     WO-A1-90/06997
WO-A1-92/05273     WO-A1-92/15676
WO-A1-93/13807     WO-A1-98/02170
WO-A1-98/19712     US-A- 5 219 740**

- **CAMPBELL A I M ET AL: "Gene transfer to the
pulmonary vasculature using genetically
engineered smooth muscle cells" JOURNAL OF
THE AMERICAN COLLEGE OF CARDIOLOGY,
ELSEVIER, NEW YORK, NY, US, vol. 31, no. 2
Suppl. A, 1 February 1998 (1998-02-01), page 25A,
XP009130127 ISSN: 0735-1097**

Remarks:
The file contains technical information submitted after
the application was filed and not included in this
specification

SEQUENCE LISTING

<110> AN-GO-GEN INC.

<120> Cell-Based Gene Therapy For the Pulmonary system

<130> G1557 EP/1 s3

<140> EP07009031
<141> 1999-09-23

<160> 6

<170> PatentIn version 3.4

<210> 1
<211> 20
<212> DNA
<213> Artificial

<220>
<223> primer sequence

<400> 1
tcgggcctcc gaaaccatga          20

<210> 2
<211> 20
<212> DNA
<213> artificial

<220>
<223> primer sequence

<400> 2
cctggtgaga gatctggttc          20

<210> 3
<211> 33
<212> DNA
<213> Artificial

<220>
<223> primer sequence

<400> 3
cgctactggc ttatcgaaat taatacgact cac          33

<210> 4
<211> 28
<212> DNA
<213> Artificial

<220>
<223> primer sequence

<400> 4
ggccttggtg aggtttgatc cgcataat          28

<210> 5
<211> 24
<212> DNA
<213> Artificial

<220>
<223> primer sequence

<400> 5
ctctaaggct gtgggcaagg tcat          24

<210> 6
<211> 24
<212> DNA
<213> Artificial

<220>
<223> primer sequence

<400> 6
gagatccacc accctgttgc tgta          24

**Description**

## FIELD OF THE INVENTION

This invention relates to medical treatments and composition and procedures useful therein. More specifically, it relates to cell-based gene transfer systems for administration to the pulmonary system of a mammalian patient. Accordingly, the invention relates to the use of viable, transfected mammalian cells selected from fibroblasts and endothelial cells, said transfected mammalian cells containing at least one expressible trans-gene coding for an angiogenic or vasoactive factor for the preparation of a pharmaceutical composition to alleviate a pulmonary disorder in a patient or symptoms thereof, wherein said pharmaceutical composition is to be administered to the pulmonary circulation of a mammalian patient.

## BACKGROUND OF THE INVENTION

Cell-based gene transfer is a known, albeit relatively new and experimental, technique for conducting gene therapy on a patient. In this procedure, DNA sequences containing the genes which it is desired to introduce into the patient's body (the trans-genes) are prepared extracellularly, e.g. by using enzymatic cleavage and subsequent recombination of DNA with insert DNA sequences. Mammalian cells such as the patient's own cells are then cultured in vitro and treated so as to take up the transgene in an expressible form. The trans-genes may be foreign to the mammalian cell, additional copies of genes already present in the cell, to increase the amount of expression product of the gene or copies of normal genes which may be defective or missing in a particular patient. Then the cells containing the transgene are introduced into the patient, so that the gene may express the required gene products in the body, for therapeutic purposes. The take-up of the foreign gene by the cells in culture may be accomplished by genetic engineering techniques, e.g. by causing transfection of the cells with a virus containing the DNA of the gene to be transferred by lipofection, by electro-poration, or by other accepted means to obtain transfected cells. This is sometimes followed by selective culturing of the cells which have successfully taken up the transgene in an expressible form, so that administration of the cells to the patient can be limited to the transfected cells expressing the trans-gene. In other cases, all of the cells subject to the take-up process are administered.

This procedure has in the past required administration of the cells containing the trans-gene directly to the body organ requiring treatment with the expression product of the trans-gene. Thus, transfected cells in an appropriate medium have been directly injected into the liver or into the muscle requiring the treatment, or via the systemic arterial circulation to enter the organ requiring treatment.

Previous attempts to introduce such genetically modified cells into the systemic arterial circulation of a patient have encountered a number of problems. For example, there is difficulty in ensuring a sufficiently high assimilation of the genetically modified cells by the specific organ or body part where the gene expression product is required for best therapeutic benefit. This lack of specificity leads to the administration of excessive amounts of the genetically modified cells, which is not only wasteful and expensive, but also increases risks of side effects. In addition, many of the transplanted genetically modified cells do not survive when administered to the systemic arterial circulation, since they encounter relatively high arterial pressures. Infusion of particulate materials, including cells, to other systemic circulations such as the brain and the heart, may lead to adverse consequences due to embolization, i.e. ischemia and even infarction.

It is an object of the present invention to provide a novel procedure of cell based gene transfer to mammals.

It is a further and more specific object of the invention to provide novel procedures of cell-based gene therapy utilizing dermal (or other) fibroblast cells.

It is a further object of the invention to provide novel genetically engineered cells containing trans-genes expressing angiogenic factors.

## SUMMARY OF THE INVENTION

The invention relates to the embodiments as defined in the claims.

The present invention is based upon the discovery that the pulmonary system of a mammal, including a human, offers a potentially attractive means of introducing genetically altered cells into the body, for purposes of gene therapy, i.e. cell based gene transfer, using fibroblasts or endothelial cells of the patient as the carriers of the trans-genes. The pulmonary system has a number of unique features rendering it particularly suited to a cell-based gene transfer. Thus, low arterial pressure and high surface area with relatively low shear in the micro-circulation of the lungs increase the chances of survival of the transplanted cells. High oxygenation in the micro-circulation of the ventilated lung also improves the viability of the transplanted cells.

Moreover, the pulmonary circulation functions as a natural filter, and is able to retain the infused cells efficiently and effectively. Also, the lung has a dual circulation (pulmonary arterial and bronchial). This is in contradistinction to other systemic circulations, such as the brain and the heart, where the infusion of particulate materials such as cells could lead to the aforementioned adverse consequences. The lung presents a massive vascular system. The high surface area of the pulmonary endothelium allows the migration of the transplanted cells trapped in the micro-circulation across the endothelial layer to take up residence within the perivascular space.

The pulmonary circulation, unlike any other circulation in the body, receives the entire output of the heart. Accordingly, it offers the greatest opportunity to release a gene product into the circulation. This distinct property of the lung is particularly useful for pulmonary gene therapy and for the treatment of a systemic disorders, as well as a pulmonary disorder.

It is believed that the transfected cells become lodged in the small artery-capillary transition regions of the pulmonary circulation system, following simple intravenous injection of the transfected cells to the patient. Products administered intravenously move with the venous circulation to the right side of the heart and then to the lungs. The transfected cells administered according to the invention appear to lodge in the small arteriolar-capillary transition regions of the circulatory system of the lungs, and then transmigrate from the intraluminal to the perivascular space, from where they deliver expression products of the trans-genes to the lungs, making the process to the present invention especially applicable to treatment of pulmonary disorders. Some factors, especially stable factors can be secreted to the general circulation for treatment of disorders of other body organs.

Thus, according to a first aspect disclosed herein, there is provided a process of conducting gene therapy in a mammalian patient, which comprises administering to the pulmonary system of the patient, genetically modified mammalian cells selected from fibroblasts and endothelial cells, said cells containing at least one expressible trans-gene which is capable of producing at least one gene product in the pulmonary circulation after administration thereto.

According to another aspect of the invention, there are provided genetically modified mammalian cells selected from fibroblasts and endothelial cells, said cells containing at least one expressible transgene coding for a therapeutic factor.

A further aspect of the present invention provides the use in the preparation of a medicament for administration to a mammalian patient to alleviate symptoms of a disorder, of viable, transfected mammalian cells selected from fibroblasts and endothelial cells, said transfected mammalian cells containing at least one expressible trans-gene coding for a therapeutic factor. Accordingly, the present invention relates to the use of ex vivo genetically modified mammalian cells selected from dermal fibroblasts and endothelial cells, containing at least one expressible trans-gene which is capable of expressing at least one gene product in the pulmonary circulation after administration thereto, said transgene being one coding for an angiogenic factor, nitric oxide synthase, a lipoprotein, a vasoactive substance, an antioxidant, a free radical scavenger, a soluble cytokine receptor, a soluble adhesion molecule, a soluble receptor for a virus or a cytokine; or the cystic fibrosis gene for the preparation of a pharmaceutical composition for conducting gene therapy in a mammalian patient, wherein said pharmaceutical composition is to be administered to the pulmonary circulation of the mammalian patient. Further, the present invention provides the use of ex vivo genetically modified dermal fibroblast cells containing an expressible trans-gene coding for an angiogenic factor for the preparation of a pharmaceutical composition comprising genetically engineered cells, for alleviating the symptoms of pulmonary hypertension in a mammalian patient suffering therefrom, wherein said pharmaceutical composition is to be administered to the pulmonary circulation of the mammalian patient. Moreover, the present invention relates to the use of viable, transfected mammalian fibroblasts containing at least one expressible cystic fibrosis trans-gene for the preparation of a pharmaceutical composition to alleviate a pulmonary disorder in a patient or symptoms thereof, wherein said pharmaceutical composition is to be administered to the pulmonary circulation of a mammalian patient. Herein provided is the use of ex vivo genetically modified mammalian fibroblasts containing at least one expressible cystic fibrosis trans-gene which is capable of expressing at least one gene product in the lung after administration thereto, for the preparation of a pharmaceutical composition for conducting gene therapy in a mammalian patient, wherein said pharmaceutical composition is to be administered to the pulmonary circulation of the mammalian patient.

Yet another aspect disclosed herein is a process of preparing genetic modifications of mammalian cells selected from fibroblasts and endothelial cells, which comprises transfecting said mammalian cells with at least one gene coding for a therapeutic factor, to produce transfected cells capable of expressing said therapeutic factor in vivo.

## DESCRIPTION OF THE PREFERRED EMBODIMENTS

A wide variety of trans-genes encoding therapeutic factors can be used in the processes and products of the present invention. While treatment of pulmonary system disorders is a primary focus of the invention, it is not limited to such treatments. Therapeutic factors expressed by the trans-genes and delivered by the circulation of other body organs downstream of the lungs are within the scope of this invention. Trans-genes expressing therapeutic factors such as Factor VIII for treatment of classical haemophelia, and other clotting factors for treating various bleeding disorders may be used. Other examples include:

trans-genes expressing hormones, for example growth hormone for treatment of hypopituitary dysfunction, insulin, (thyroid stimulating hormone (TSH) for treatment hypothyroidism following pituitary failure, and other hormones;
trans-genes expressing beneficial lipoproteins such as Apo 1 and other proteins/enzymes participating in lipid metabolism such as lipoprotein lipase;
trans-genes expressing prostacyclin and other vasoactive substances;
trans-genes expressing anti-oxidants and free radical scavengers;
trans-genes expressing soluble cytokine receptors to neutralize actions of damaging levels of immune mediators, for example soluble TNFα receptor, or cytokine receptor antagonists, for example IL1ra;
trans-genes expressing soluble adhesion molecules, for example ICAM-1, to interrupt pathological cell adhesion processes such as those which occur in inflammatory diseases;
trans-genes expressing soluble receptors for viruses to inhibit infection of cells, e.g. CD4, CXCR4, CCR5 for HIV;
trans-genes expressing cytokines, for example IL-2, to activate immune responses for combating infections;

the cystic fibrosis gene, as a trans-gene.

The transfected cells lodged in the lung and containing trans-genes expressing such factors and other products will act as a systemic source of the appropriate factor.

One preferred aspect of the present invention is the treatment of pulmonary hypertension (PH). Primary pulmonary hypertension (PPH) and other causes of PH are associated with severe abnormalities in endothelial function, which likely play a critical role in its pathogenesis. The vasoditatory, antithrombotic and anti-proliferative factor, nitric oxide (NO) has been demonstrated to decrease pulmonary pressures in both experimental and clinical situations. However, long-term viral-based methods may cause significant local inflammation. Other, previous attempts to treat PPH have involved the use of prostacyclin, using continuous administration, but this is a difficult and expensive procedure, liable to give rise to side effects.

The present invention provides, from this second preferred aspect, the use of transformed mammalian fibroblast cells from dermal or other origins, said transfected cells including at least one expressible trans-gene coding for an angiogenic factor for release thereof into the pulmonary circulation for the preparation of a pharmaceutical composition to alleviate the symptoms of PPH (and other causes of PH), wherein the pharmaceutical composition is to be administered to the pulmonary system of a patient suffering therefrom.

Specific examples of useful angiogenic factors in this preferred aspect of the present invention include nitric oxide synthase (NOS); vascular endothelial growth factor (VEGF) in all of its various known forms, i.e. VEGF165 which is the commonest and is preferred for use herein, VEGF205, VEGF189, VEGF121, VEGFB and VEGFC (collectively referred to herein as VEGF); fibroblast growth factor (FGF, acid and basic), angiopoietin-1 and other angiopoietins, transforming growth factor -β (TGF-β), and hepatic growth factor (scatter factor) and hypoxia inducible factor (HIF). DNA sequences constituting the genes for these angiogenic factors are known, and they can be prepared by the standard methods of recombinant DNA technologies (for example enzymatic cleavage and recombination of DNA), and introduced into mammalian cells, in expressible form, by standard genetic engineering techniques such as those mentioned above (viral transfection, electroporation, lipofection, use of polycationic proteins, etc). VEGF is the preferred angiogenic factor, on account of the greater experience with this factor and its level of effective expression in practice.

Angiogenic factors such as those mentioned above have previously been proposed for use as therapeutic substances in treatment of vascular disease. It is not to be predicted from this work, however, that such angiogenic factors would also be useful in treatment of pulmonary hypertension. Whilst it is not intended that the scope of the present invention should be limited to any particular theory or mode of operation, it appears that angiogenic growth factors may also have properties in addition to their ability to induce new blood vessel formation. These other properties apparently include the ability to increase nitric oxide production and activity, and/or decrease the production of endothelin-1, in the pulmonary circulation, so as to improve the balance of pulmonary cell nitric oxide or endothelin-1 production.

In preparing cells for transfection and subsequent introduction into a patient's pulmonary system, it is preferred to start with dermal fibroblasts or endothelial cells obtained from the eventual recipient of the cell-based gene transfer treatment of the present invention. Dermal fibroblasts are simply and readily obtained from the patient's exterior skin layers, readied for in vitro culturing by standard techniques. Endothelial cells are harvested from the eventual recipient, e.g. by removal of a saphenous vein and culture of the endothelial cells. The culture methods are standard culture techniques with special precautions for culturing of human cells with the intent of re-implantation.

It is strongly preferred, in accordance with the present invention, to use dermal fibroblasts from the patient, as the cells for gene transfer. Given the fact that the logical choice of cell types for one skilled in the art to make would be a cell type naturally found in the patient's pulmonary system, such as smooth muscle cells, the use of fibroblasts is counter-intuitive. Surprisingly, it has been found that fibroblasts are eminently suitable for this work, exhibiting significant and unexpected advantages over cells such as smooth muscle cells. They turn out to be easier to grow in culture, and easier to transfect with a transgene, given the appropriate selection of technique. They yield a higher proportion of transfectants, and a higher degree of expression of the angiogenic factors in vivo, after introduction into the patient's pulmonary system. The anticipated greater risk with fibroblasts of possibly causing fibrosis in the pulmonary system, as compared with smooth muscle cells, has not materialized.

The somatic gene transfer in vitro to the recipient cells, i.e. the genetic engineering, is performed by standard and commercially available approaches to achieve gene transfer, as outlined above. Preferably, the method includes the use of poly cationic proteins (e.g. SUPERFECT*) or lipofection (e.g. by use of GENEFECTOR), agents available commercially and which enhance gene transfer. However other methods besides lipofection and polycationic protein use, such as, electroporation, viral methods of gene transfer including adeno and retro viruses, may be employed. These methods and techniques are well known to those skilled in the art, and are readily adapted for use in the process of the present invention. Lipofection is the most preferred technique, for use with dermal fibroblast host cells.

The re-introduction of the genetically engineered cells into the pulmonary circulation can be accomplished by infusion of the cells either into a peripheral vein or a central vein, from where they move with the circulation to the pulmonary system as previously described, and become lodged in the smallest arterioles of the vascular bed of the lungs. Direct injection into the pulmonary circulation can also be adopted, although this is less convenient. The infusion can be done either in a bolus form i.e. injection of all the cells during a short period of time, or it may be accomplished by a continuous infusion of small numbers of cells over a long period of time, or alternatively by administration of limited size boluses on several occasions over a period of time.

While the transfected cells themselves are largely or completely retained in the pulmonary circulation, and especially in the arterioles of the patient's lungs, the expression products of the trans-genes thereof are not restricted in this manner. They can be expressed and secreted from the transfected cells, and travel through the normal circulation of the patient to other, downstream body organs where they can exert a therapeutic effect. Thus, while a preferred use of the process of the invention is in the treatment of pulmonary disorders, since the express ion products initially contact the patient's pulmonary system, it is not limited to such treatments. The transfectants can contain trans-genes expressing products designed for treatment of other body organs of the patient. Such products expressed in the pulmonary system will target the other, predetermined organs and be delivered thereto by the natural circulation system of the patient.

The invention is further described for illustrative purposes, in the following specific, non-limiting Examples.

## EXAMPLE 1 - SKIN FIBROBLAST EXPLANT CULTURE

Fisher 344 rats (Charles River Co.) were obtained at 21 days of age and were sacrificed by overdose with ketamine and xylazine. The hair was carefully shaved and the back skin was excised and transferred immediately into a phosphate-buffered saline (PBS) solution containing 2% penicillamine and streptomycin (Gibco BRL, Burlington, Ontario). The epidermal and deep fat and connective tissue was removed using a scalpel. The dermal tissue was cut into approximately 4 millimetre square pieces which were placed on individual fibronectin-coated (Sigma Chemical Co., Mississauga, Ontario) tissue culture plates (Falcon, Becton Dickinson Canada, Mississauga, Ontario). The explants were then grown in Dulbecco's Modified Eagle Media with 20% fetal calf serum (FCS) and 2% penicillamine and streptomycin (all Gibco BRL), in a humidified environment with 95% 02 and 5% CO2 at 37 °C, with the media being changed every second day. Explants were passaged using 0.05% trypsin/EDTA (Gibco BRL) once many thin, spindle-shaped cells could clearly be seen growing from the dermal explant and the remaining explanted tissue was removed. The cells were then grown in DMEM with 20% FCS and 2% penicillamine and streptomycin until they were to be used in further experiments.

The purity of the cells as to effective type was checked, using antibodies and standard staining techniques, to determine the approximate number of available, effective cells of fibroblast lineage.

## EXAMPLE 2 - FLUORESCENT CELL LABELING

Cells between the fifth and ninth passages were grown until 80% confluent and were then labelled with the fluorophore, chloromethyl trimethyl rhodamine (CMTMR, Molecular Probes Inc., Eugene, Oregon). CMTMR affords a very accurate method of detecting *ex vivo* labelled cells, as the molecule undergoes irreversible esterification and glucoronidation after passing into the cytoplasm of a cell and thereby generates a membrane-impermeable final product. This active fluorophore is thus unable to diffuse from the original labelled cell into adjacent cells or structures, and may be detected in vivo for several months, according to the manufacturer. The fluorescent probe was prepared by dissolving the lyophilized product in dimethyl sulfoxide (DMSO) to a concentration of 10 millimolar. This solution was stored at 20 degrees C and diluted to a final concentration of 25 micromolar in serum-free DMEM immediately prior to use. Cells were exposed to the labelling agent for 45 minutes, and were then washed with PE S twice and the regular media (DMEM with 10% FCS and 2% penicillin and streptomycin) replaced. The cells were grown overnight and harvested 24 hours later for injection into the internal jugular vein of recipient Fisher 344 rats.

A series of in vitro experiments was also performed by plating the cells on cover slips and the incubating them with the fluorophore to determine the quality and duration of fluorescence over time. Immediately after incubation with the fluorophore, CMTMR, at a concentration of 25 micromolar, 100% of cultured cells were found to fluoresce ir tensely when examined under a rhodamine filter. Cells were also examined 48 hours and 7 days after labelling, and despite numerous cell divisions 100% of the cells present on the cover slip continued to fluoresce brightly (data not shown).

EXAMPLE 3 - *EX VIVO* CELL TRANSFECTION WITH THE CMV- βGal PLASMID

The vector CMV- βGal (Clontech Inc., Palo Alto, California), which contains the beta-galactosidase gene under the control of the cytomegalovirus enhancer/promoter sequence, was used as a reporter gene to follow the course of in vivo transgene expression. Fibroblasts were grown to 70 to 80% confluence. The optimal ratio of liposome to DNA was determined to be 6 μg of liposome / 1 μg of DNA. Cells were washed with DMEM medium (no additives) and 6.4 mls of DMEM was added to each 100 mm plate. 200 μl of Genefector (Vennova Inc., Pablo Beach Fla) was diluted in 0.8 mls of DMEM and mixed with 16 μg of DNA (CMV- βgal) also diluted in 0.8 ml of DMEM. The liposome solution was then added dropwise over the entire surface of the plate, which was gently shaken and incubated at 30°C for eight hours. This method was used to avoid the use of viral vectors and simultaneously obtain significant in vitro transfection efficiencies. The Genefector product is an optimized liposome preparation. This Genefector-DNA complex then interacts with cell surface and is transported into the cytoplasm, after which the plasmid DNA can translocate to the nucleus.
Then the transfection medium was replaced with 20% FBS, with 2% penicillin/streptomycin in M199 media and incubated for 24 to 48 hours. This method resulted in transfection efficiencies between 40 and 60%.

EXAMPLE 4 - ANIMAL SURGERY AND DETECTION OF FLUORESCENTLY-LABELED CELLS IN TISSUE

All animal procedures were approved by the Animal Care Committee of St. Michael's Hospital, Toronto, Canada. Six week old Fisher 344 rats (Charles River Co., St. Constant, Quebec) were anesthetized by intraperitoneal injection of xylazine (4.6 milligrams/kilogram) and ketamine (70 milligrams/kilogram), and the cervical area shaved and cleaned with iodine and ethanol. A midcervical incision was made with a scalpel and the right internal, external and common jugular veins identified. Plastic tubing of 0.02 millimetres external diameter was connected to a 23 gauge needle and flushed with sterile saline (Baxter). Thus tubing was then used to cannulate the external jugular vein and was introduced approximately 5 centimetres into the vein to what was estimated to be the superior vena cava level, and rapid venous blood return was used to confirm the catheter location.
For experiments to determine the time course of cell survival and transgene expression in the lung, dermal fibroblasts cells which had been labelled with the fluorophore CMTMR, or transfected with the plasmid vector CMV- βGal, were trypsinized, and centrifuged at 850 rpm for 5 minutes. The excess medium was removed and the pellet of cells was resuspended in a total volume of 2 millilitres of phosphate-buffered saline (PBS). A 50 microlitre aliquot of these resuspended cells was then taken and counted on a hemocytometer grid to determine the total number of cells present per millilitre of PBS. The solution was then divided into 1 millilitre aliquots of approximately 500,000 cells and transferred in a sterile manner to the animal care facility. These cells were then resuspended by gentle vortexing and injected into the animals via the external jugular vein catheter. The solution was infused slowly over one to two minutes and the catheter was then flushed again with sterile saline prior to removal. The external jugular vein was ligated, the incision closed with 3-0 interrupted absorbable sutures, and the animals allowed to recover from surgery.
At 30 minutes or 24 hours after delivery of labelled cells (n=3 for each time-point), or saline injection (negative control, n=3), the animals were sacrificed by anesthetic overdose, and the chest cavity was opened. The pulmonary artery and trachea were flushed with saline, and the right and left lungs excised. Transverse slices were taken from the basal, medial and apical segments of both lungs, and specimens obtained from the liver, spleen, kidney and gastronemius muscle. Tissue specimens were embedded in OCT compound (Sakura Finetek U.S.A. Inc. Torrance, California) en face, and then flash frozen in liquid nitrogen. Ten micron sections were cut from these frozen blocks at 2 different tissue levels separated by at least 200 microns, and these sections were then examined under a fluorescent microscope using a rhodamine filter, and the number of intensely fluorescing cells was counted in each en face tissue specimen.
To provide an estimate of the total number of labelled cells present within the entire lung, the total number of fluorescent cells were counted in each lung section and averaged over the number of sections counted. A mathematical approximation could be made of the total number of cells present within the lung by utilizing Simpson's rule for the volume of a truncated cone. This equation bases the total volume of a cone on the relative areas of 3 different sections such that:

$$\text{volume} = [(\text{area}^{basal\ section} + \text{area}^{middle\ section} \times \text{height of the lung}]/3 + [\text{area}^{apical\ section}/2 \times \text{height of the lung}/3] + [\lambda/6 \times (\text{height of the lung}/3)^3].$$

The height of the lung was measured after organ harvesting, and the area of each transverse section was determined by planimetry. The average number of cells present in the three sections, divided by the total volume of these sections yielded an estimate of the cell number per unit volume. By multiplying this number by the total lung volume an estimate of the total number of cells within the lung could be obtained. To correct for the appearance of a single cell in multiple adjacent lung sections, rats were injected with 500,000 CMTMR labelled cells and sacrificed acutely. The lungs were prepared, harvested and embedded in the usual manner, and twenty serial sections, each 5 microns in thickness, were taken through the lung parenchyma. Each section was examined using a rhodamine filter and distinct individual cells were identified and their presence determined on adjacent sections. The number of 5 micron sections in which a single cell could be identified was counted and the average dimensions of a pulmonary artery smooth muscle cell in vivo was obtained. The average diameter observed was $16.4 \pm 1.22$ microns. Therefore, the total number of cells calculated using the Simpson's formula was multiplied by 0.61 to correct for the presence of 1 cell in, on average, each 1.64 ten micron sections.

30 minutes after fibroblast delivery, $373 \pm 36$ CMTMR-labelled cells /$cm^2$ were identified within the lung sections, which represented approximately 60% of the total number of cells injected. After 24 hours there was only a slight decrease in CMTMR-labelled cells to $317 \pm 4/cm^2$ or 85% of the 30-minute value, indicating excellent survival of transplanted cells. The survival of CMTMR-labelled cells at later time points of 2, 4, 7, and 14 days and 1, 2, 3 and 6 months are also evaluated to establish the time course of transplanted cell survival in the lungs of recipient rats. No brightly fluorescent signals were seen in any of the lungs injected with non-labelled smooth muscle cells.

In the spleen, liver and skeletal muscle tissue no fluorescent signals were identified. In 2 out of 4 kidneys examined, irregular fluorescent signals could be identified. None of these appeared to conform to the shape of a whole cell, and were presumed to represent those cells that were sheared or destroyed during cell injection or short y thereafter. In addition, no fluorescent signals were identified in any organ outside of the lung 7 days after injection.

EXAMPLE 5 - DETECTION OF BETA-GALACTOSIDASE EXPRESSION IN TISSUE

At various time-points after cell-based gene transfer, animals are sacrificed and the chest opened. The pulmonary artery is flushed with saline and the trachea is cannulated and flushed with 2% paraformaldehyde until the lungs are well inflated. Transverse slices are taken from the basal, medial and apical segments of both lungs, and specimens obtained from the liver, spleen, kidney and gastroenemius muscle of certain animals. The specimens are incubated in 2% paraformaldehyde with 0.2% glutaraldehyde for 1 hour, and then rinsed in PBS. The tissue is then incubated for 18 hours at 37°C with a chromogen solution containing 0.2% 5-bromo-4-chloro-3-indolyl-$\beta$-D-galactoside (X-Gal, Boehringer Mannheim, Laval, Quebec), 5 millimolar potassium ferrocyanide (Sigma), 5 millimolar potassium ferricyanide (Sigma), and 2 millimolar magnesium chloride (Sigma), all dissolved in phosphate buffered saline. The specimens are the rinsed in PBS, embedded in OCT compound (Miles Laboratories), cut into 10 micron sections, and counterstained with neutral red.

Then face sections are examined microscopically, and the number of intensely blue staining cells can be estimated. As one dish of cells is used for in vitro staining to determine the transfection efficiency for each reaction series, an estimate of the percentage of cells that were transfected with the reporter gene plasmid pCMV-$\beta$Gal can be made for every animal. Using this information and the mathematical calculation described for approximating the number of fluorescent t cells present, an estimate can be made of the total number of transfected cells remaining at the time of animal sacrifice.

In a number of separate transfection reactions using the pCMV-$\beta$Gal plasmid, an average transfection efficiency of the order of 40 - 50% can be obtained with the dermal fibroblasts in vitro. No staining is seen in mock transfected cultures.

Following incubation with the X-Gal chromogen solution, microscopic evidence of cell-based transgene expression can be seen at 48 hours after injection of pCMV-$\beta$Gal transfected dermal fibroblast cells into the internal jugular vein, with multiple intense blue staining cells being seen throughout the lung, representing at least 30% of the original transfected cells that were injected. As with the fluorescently-labeled cells, most of the beta-galactosidase expressing cells appear to be lodged within the distal microvasculature of the lung. No evidence of beta-galactosidase expression is detected in any of the lungs from animals injected with non-transfected dermal fibroblast cells. No evidence of pulmonary pathology, as determined by the presence of an abnormal polymorphonuclear or lymphocytic infiltrate, septal thickening or alveolar destruction, is observed.

EXAMPLE 6 - MONOCROTALINE PREVENTION STUDIES

To determine if cell-base gene transfer of VEGF165 would be capable of inhibiting the development of pulmonary hypertension in an animal model of the disease, dermal fibroblasts which had been transfected with either pVEGF or pcDNA 3.1 (an empty vector) were prepared as described above. The full-length coding sequence of $VEGF_{165}$ was generated by performing a reverse transcription polymerase chain reaction using total RNA extracted from human aortic smooth muscle cells and the following sequence specific primers: sense 5' TCGGGCCTCCGAAACCATGA 3', antisense 5' CCTGGTGAGAGATCTGGTTC 3'. This generated a 649 bp fragment which was cloned into the pGEM-T vector (Promega, Madison, Wisconsin), and sequenced to confirm identity. The fragment was then cloned into the expression vector pcDNA 3.1 at the EcoR1 restriction site, and correct orientation determined using a differential digest. The insert deficient vector (pcDNA 3.1) was used as a control for the monocrotaline experiments. All plasmid DNA was introduced into a JM 109 strain of *E. Coli* via the heat-shock method of transformation, and bacteria were cultured overnight in LB media containing 100 micrograms/millilitre of ampicillin. The plasmid was then purified using an endotoxin-free purification kit according to the manufacturer's instructions (Qiagen Endotoxin-Free Maxi Kit, Qiagen Inc., Mississauga, Ontario), producing plasmid DNA with an $A_{260}/A_{280}$ ratio of greater than 1.75, and a concentration of at least 1.0 micrograms/microliter.

Transfected dermal fibro blasts were trypsinized and divided into aliquots of 500,000 cells. Six to eight week old Fisher 344 rats were then anesthetized and injected subcutaneously with either 80 milligrams/kilogram of monocrotaline (n=13) (Aldrich Chemical Co., Milwaukee, Wisconsin) alone, or with monocrotaline and, via a catheter n the external jugular vein, either 500,000 pVEGF (n=5), or pcDNA 3.1 (n=3) transfected cells.

Monocrotaline is a plant alkaloid, a metabolite of which damages the pulmonary endothelium, providing an animal model of pulmonary hypertension.

The vein was tied off, the incision closed in the normal fashion, and the animals allowed to recover. At 28 days after injection, animals were reanesthetized, and a Millar microtip catheter reinserted via the right internal jugular vein into the right ventricle. The right ventricular systolic pressure was recorded, and the catheter was then inserted into the ascending aorta and the systemic arterial pressure recorded. The animals were then sacrificed and the hearts excised. The right ventricular (RV) to left ventricular plus septal (LV) weight ratios (RV/LV ratio) were determined as an indicator of hypertrophic response to long-standing pulmonary hypertension. Lungs were flushed via the pulmonary artery with sterile phosphate-buffered saline, and were gently insufflated with 2% paraformaldehyde via the trachea. Pulmonary segments were then either snap frozen in liquid nitrogen for subsequent RNA extraction, or were fixed via immersion in 2% paraformaldehyde for paraffin embedding and sectioning. The right ventricular systolic pressures and RV/LV ratios were compared between the pVEGF, pcDNA 3.1, and monocrotaline alone groups.

RNA extracted from rat lungs was quantified, and 5 micrograms of total RNA from each animal was reverse transcribed using the murine moloney leukemia virus reverse-transcriptase, and an aliquot of the resulting cDNA was amplified with the polymerase chain reaction (PCR) using the following sequence-specific primers: sense 5' CGCTACTGGCTTATCGAAATTAATACGACTCAC 3', antisense 5' GGCCTTGGTGAGGTTTGATCCGCATAAT 3', for 30 cycles with an annealing temperature of 65 °C. Ten microlitres of a fifty microlitre reaction were run on a 1.5% agarose gel. The upstream primer was located within the T7 priming site of the pcDNA 3.1 vector and therefore should not anneal with any endogenous RNA transcript, and the downstream primer was located within exon 4 of the coding region of VEGF. Therefore, the successful PCR reaction would selectively amplify only exogenous VEGF RNA. To control for RNA quantity and quality a second aliquot of the same reverse transcription reaction was amplified with the following primers for the constitutively-expressed gene GAPDH: sense 5' CTCTAAGGCTGTGGGCAAGGTCAT 3', antisense 5' GAGATCCACCACCCTGTTGCTGTA 3'. This reaction was carried out for 25 cycles with an annealing temperature of 58°C. Ten microlitres of a fifty microlitre reaction were run on a 1.5% agarose gel, and compared to the signal obtained from the VEGF PCR.

Paraformaldehyde fixed rat lungs were cut perpendicular to their long axis and were paraffin-embedded en face. Sections were obtained and stained using the elastin-von Giessen's (EVG) technique. The sections were assessed by a blinded observer who measured all vessels with a perceptible media within each cross-section under 40X magnification using the C+ computer imaging system. The medial area of each vessel was determined and an average was obtained for each vessel size from 0 to 30, 30 to 60, 60 to 90, 90 to 120, and greater than 120 microns in external diameter, for each animal. The averages from each size were compared between the pVEGF, pcDNA 3.1, and monocrotaline alone groups.

Four weeks following monocrotaline injection (n=11) alone, the right ventricular systolic pressure was increased to $48 \pm 2$ mm Hg, and there was no improvement in those animals who received the pcDNA 3.1 transfected cells (n=3) with the average RVSP remaining at $48 \pm 2$ mm Hg. However, in those animals treated with the pVEGF transfected fibroblasts (n=5) the RV pressure was significantly decreased to $32 \pm 2$ m n Hg (p<0.0001).

As anticipated from the long-standing pulmonary hypertension, the RV/LV ratio was significantly elevated from baseline following monocrotaline injection (n=13) to 0.345 0.015 and was very similar in the pcDNA 3.1 transfected group (n=3). Following VEGF gene transfer (n=5) the ratio was significantly lower than the pcDNA 3.1 transfected group. No difference in aortic pressure was noted. Four weeks after injection of the pulmonary endothelial toxin monocrotaline and transfected cells, the RV/LV ratio is significantly elevated in the MCT group and in the pcDNA 3.1 group, but was lower in the pVEGF transfected animals.

Morphometric analysis of the tissue sections revealed that in both the monocrotaline alone and the pcDNA 3.1 treated groups, the medial area for the vessel groups from 0 to 30, 30 to 60 and 60 to 90 microns was significantly increased, as compared to the VEGF treated animals. Similar results were seen in animals transfected with the control vector, pcDNA 3.1. Following cell-based gene transfer of VEGF, a significant decrease in medial thickness and area was observed in vessels of 0 to 90 microns external diameter.

Using the plasmid-based primers, the exogenous VEGF transcript was selectively amplified using the polymerase chain reaction. RNA quality and loading was assessed by amplifying the house-keeping gene GAPDH, which was consistently present in all samples. This demonstrated that the foreign RNA was being transcribed 28 days after cell-based gene transfer and that potentially the presence of the transcript, and presumably the translated protein, was causally related to the lowering of RVSP in the VEGF treated animals.

Blood taken from the animals by left ventricular puncture immediately before sacrifice was analyzed for pH, oxygen loading (p02), carbon dioxide loading (pCO2) and % saturation. The results are given below.

## BLOOD ANALYSIS DATA

VEGF/fibroblast transfected animals

|  | pH | pCO2 | pO2 | %Sat'n |
|---|---|---|---|---|
| Mean (of 5 animals) | 7.374 | 51.2 | 78.3 | 86.58 |
| Standard deviation, SD | 0.0502 | 5.699 | 17.89 | 9.867 |

pcDNA/fibroblast transfected animals

|  | pH | pCO2 | pO2 | %Sat'n |
|---|---|---|---|---|
| Mean (of 3 animals) | 7.35 | 58.333 | 60.8 | 74.4 |
| SD | 0.02 | 3.761 | 7.615 | 7.882 |

These results indicate preliminarily that arterial $O_2$ tension and saturation are better in the VEGF transfected group than in animals receiving null-transfected cells. This is consistent with the improvement in pulmonary hemodynamics and lung vascular morphology, and argues against significant right to left shifting as might occur in pulmonary arterial to venous shunts.

The creation of "shunting" - formation of new passageways between the arteries and the capillaries of the pulmonary system, by-passing the veins and thereby limiting the blood oxygen up-take, does not occur to any problematic extent, according to prelim nary indications.

EXAMPLE 7 - MONOCROTALINE REVERSAL STUDIES

To determine if dermal fibroblast cell-based gene transfer of VEGF 165 would be capable of reversing or preventing the progression of established pulmonary hypertension in an animal model of disease, six to eight week old Fisher 344 rats are injected subcutaneously with 80 milligrams/kilogram of monocrotaline. Fourteen days after monocrotaline injection the animals are anesthetized and a Millar catheter is passed into the right ventricle and the RV pressure recorded. Dermal fibroblast cells transfected with either pVEGF or pcDNA 3.1 are then injected in aliquots of 500,000 cells into the external jugular vein, and the animals allowed to recover. At 28 days after monocrotaline injection and 14 days after cell-based gene transfer, the animals are reanesthetized, and a Millar microtip catheter reinserted via the right internal jugular vein into the right ventricle. The right ventricular systolic pressure (RVSP) is recorded, and the catheter is then inserted into the ascending aorta and the systemic arterial pressure recorded. The animals are then sacrificed and the hearts excised. The RV/LV ratios are determined as an indicator of hypertrophic response to long-standing pulmonary hypertension. When the right ventricular systolic pressures and RV/LV ratios are compared between the pVEGF and pcDNA 3.1 groups, it is found, two weeks after monocrotaline injection, the RVSP is elevated, and in the animals who received pcDNA 3.1 transfected cells the pressure is further increased to 55 5 mm Hg at four weeks after monocrotaline delivery. However, in the pVEGF treated animals the RVSF only increases a small amount. Four weeks after injection of the pulmonary endothelial toxin monocrotaline, the RVSP increases significantly in the pcDNA 3.1 group, but is significantly decreased in the pVEGF transfected animals.

The RV/LV ratio was significantly elevated in the pcDNA group, but following VEGF gene transfer the ratio was significantly reduced. No difference in aortic pressure is observed. Four weeks after injection of monocrotaline, the ratio is increased further in the pcDNA 3.1 group, but is significantly decreased in the pVEGF transfected animals.

DISCUSSION

The present invention represents evidence of successful non-viral gene transfer to the pulmonary vasculature using transfected fibroblasts, and provides a demonstration of potential therapeutic efficacy of an angiogenic strategy in the treatment of PH using this approach. This method of delivery was associated with a high percentage of cells being retained within the lung at 48 hours, as determined by both the fluorescence labelling technique and by the reporter gene studies using beta-galactosidase, and with moderate but persistent gene expression over 14 days. These results roughly parallel what has previously been demonstrated with a viral-based method of intravascular gene delivery to the pulmonary vasculature (see Schachtner, S.K., J.J. Rome, R.F. Hoyt, Jr., K.D. Newman, R. Virman , D.A. Dichek, 1995.In vivo adenovirus-mediated gene transfer via he pulmonary artery of rats. Circ. Res. 76:701-709; and Rodman, D.M., H. San, R. Simari, D. Stephan, F. Tanner, Z. Yang, G.J. Nabel, E.G. Nabel, 1997.In vivo gene delivery to the pulmonary circulation in rats: transgene distribution and vascular inflammatory response. Am. J. Respir. Cell Mol. Biol. 16:640-649).

However, the cell-based technique provided by the present invention avoids the use of a potentially immunogenic viral construct, was not associated with any significant pulmonary or systemic inflammation, and permits more selective transgene expression within the pulmonary microvasculature, and in particular the targeting of transgene expression localized to the distal pulmonary arteriolar region, which is primarily responsible for determining pulmonary vascular resistance, and therefore PH.

The present invention addresses several key questions related to the feasibility of a cell-based gene transfer approach for the pulmonary circulation, including the survival of genetically engineered cells and the selectivity of their localization and transgene expression within the lungs. Implanted cells were efficiently retained by the lungs.

The finding that most of the cells appeared to lodge within small pulmonary arterioles is consistent with the normal physiological role the lung plays as an anatomical filter, and thus it would be expected that relatively large particles such as resuspended cells would become lodged within the pulmonary microvasculature. However, this 'targeting' of cells to the pre-capillary resistance vessel bed in a highly selective manner may prove very useful in the treatment for certain pulmonary vascular disorders. The overexpression of a vasoactive gene at the distal arteriolar level could provide a highly localized effect in a vascular region critical in the control of pulmonary vascular resistance and could amplify the biological consequences of gene transfer. In fact, the localized reduction in RVSP seen in monocrotaline-treated animals receiving VEGF transfected cells, occurred without a corresponding decrease in systemic pressures, highlighting the specificity of this method of transfection. This approach may therefore offer significant advantages over other pulmonary selective gene transfer strategies such as endotracheal gene delivery, which results in predominantly bronchial over expression, or catheter-based pulmonary vascular gene transfer, which produces diffuse macrovascular and systemic overexpression (see Rodman, D.M., H. San, R. Simari, D. Stephan, F. Tanner, Z. Yang, G.J. Nabel, E.G. Nabel, 1997. In vivo gene delivery to the pulmonary circulation in rats: transgene distribution and vascular inflammatory response. Am. J. Respir. Cell Mol. Biol 16:640-649; and Nabel, E.G., Z. Yang, D. Muller, A.E. Chang, X. Gao, L. Huang, K.J. Cho, G.J. Nabel, 1994.Safety and toxicity of catheter gene delivery to the pulmonary vasculature in a patient with metastatic melanoma. Hum. Gene Ther. 5:1089-1094).

This significant effect occurred despite an overall relatively low mass of organ-specific transfection, an d was likely due to the fact that the transfected cells were targeted, based on their size, to the precapillary pulmonary resistance vessels which play a critical role in controlling pulmonary pressure. This method of pulmonary vascular gene transfer may have benefits over existing techniques by minimizing the overall "load" of foreign transgene that is delivered to the body and may thereby theoretically reduce the incidence of undesired side-effects.

**Claims**

1.  Use of viable, transfected mammalian cells selected from fibroblasts and endothelial cells, said transfected mammalian cells containing at least one expressible trans-gene coding for an angiogenic or vasoactive factor for the preparation of a pharmaceutical composition to alleviate a pulmonary disorder in a patient or symptoms thereof, wherein said pharmaceutical composition is to be administered to the pulmonary circulation of a mammalian patient.

2.  The use according to claim 1, wherein the mammalian cells are dermal fibroblasts.

3.  The use according to claim 1 or 2, wherein the angiogenic factor is nitric oxide synthase, vascular endothelial growth factor, fibroblast growth factor, angiopoietin-1, transforming growth factor-$\beta$, hepatic growth factor (scatter factor) or hypoxia inducible factor (HIF).

4.  The use according to any one of claims 1 to 3, wherein the angiogenic factor is vascular endothelial growth factor, VEGF.

5.  The use according to any preceding claim, wherein the mammalian cells originate with the eventual recipient of the transfected cells.

6.  The use according to any preceding claim, wherein the pulmonary disorder is pulmonary hypertension.

7.  Use of ex vivo genetically modified mammalian cells selected from dermal fibroblasts and endothelial cells, containing at least one expressible trans-gene which is capable of expressing at least one gene product in the pulmonary circulation after administration thereto, said trans-gene being one coding for an angiogenic factor, nitric oxide synthase, a lipoprotein, a vasoactive substance, an antioxidant, a free radical scavenger, a soluble cytokine receptor, a soluble adhesion molecule, a soluble receptor for a virus or a cytokine; or the cystic fibrosis gene for the preparation of a pharmaceutical composition for conducting gene therapy in a mammalian patient, wherein said pharmaceutical composition is to be administered to the pulmonary circulation of the mammalian patient.

8.  The use of claim 7, wherein the genetically modified cells are dermal fibroblast cells.

9.  The use of claim 7 or 8, wherein the expressible trans-gene is a gene coding for an angiogenic factor.

10. The use of any one of claims 7 to 9, wherein the expressible gene is a gene coding for an angiogenic factor selected from nitric oxide synthase, a vascular endothelial growth factor, fibroblast growth factor, angiopoietin-1, transforming growth factor-beta and hepatic growth factor.

11. The use of claim 10, wherein the angiogenic factor is vascular endothelial growth factor, VEGF.

12. Use of ex vivo genetically modified dermal fibroblast cells containing an expressible trans-gene coding for an angiogenic factor for the preparation of a pharmaceutical composition comprising genetically engineered cells, for alleviating the symptoms of pulmonary hypertension in a mammalian patient suffering therefrom, wherein said pharmaceutical composition is to be administered to the pulmonary circulation of the mammalian patient.

13. Use of viable, transfected mammalian fibroblasts containing at least one expressible cystic fibrosis trans-gene for the preparation of a pharmaceutical composition to alleviate a pulmonary disorder in apatient or symptoms thereof, wherein said pharmaceutical composition is to be administered to the pulmonary circulation of a mammalian patient.

14. The use according to claim 13 , wherein the mammalian cells originate with the eventual recipient of the transfected cells.

15. Use of ex vivo genetically modified mammalian fibroblasts containing at least one expressible cystic fibrosis transgene which is capable of expressing at least one gene product in the lung after administration thereto, for the preparation of a pharmaceutical composition for conducting gene therapy in a mammalian patient, wherein said pharmaceutical composition is to be administered to the pulmonary circulation of the mammalian patient.


**Patentansprüche**

1. Verwendung von lebensfähigen, transfizierten Säugerzellen, ausgewählt aus Fibroblasten und Endothelzellen, wobei die transfizierten Säugerzellen zumindest ein exprimierbares Transgen enthalten, das einen angiogenen oder vasoaktiven Faktor codiert, für die Herstellung eines Arzneimittels zur Linderung einer Lungenkrankheit oder Symptomen davon bei einem Individuum, wobei das Arzneimittel direkt in den Lungenkreislauf eines Säugerindividuums zu verabreichen ist.

2. Verwendung nach Anspruch 1, wobei die Säugerzellen Hautfibroblasten sind.

3. Verwendung nach Anspruch 1 oder 2, wobei der angiogene Faktor Stickoxidsynthase, vaskulärer endothelialer Wachstumsfaktor, Fibroblasten-Wachstumsfaktor, Angiopoietin-1, Transformierender Wachstumsfaktor-$\beta$, hepatischer Wachstumsfaktor (Scatterfaktor) oder Hypoxie-induzierbarer Faktor (HIF) ist.

4. Verwendung nach einem der Ansprüche 1 bis 3, wobei der angiogene Faktor vaskulärer endothelialer Wachstumsfaktor, VEGF, ist.

5. Verwendung nach einem vorhergehenden Anspruch, wobei die Säugerzellen von dem späteren Empfänger der transfizierten Zellen stammen.

6. Verwendung nach einem vorhergehenden Anspruch, wobei die Lungenkrankheit Lungenhochdruck ist.

7. Verwendung von ex vivo genetisch veränderten Säugerzellen, ausgewählt aus Hautfibroblasten und Endothelzellen, die zumindest ein exprimierbares Transgen enthalten, das mindestens ein Genprodukt nach Verabreichung im Lungenkreislauf exprimieren kann, wobei das Transgen einen angiogenen Faktor, Stickoxidsynthase, ein Lipoprotein, eine vasoaktive Substanz, ein Antioxidans, einen freien Radikalfänger, einen löslichen Cytokinrezeptor, ein lösliches Adhäsionsmolekül, einen löslichen Rezeptor für ein Virus oder ein Cytokin oder das Cystische Fibrose-Gen codiert, für die Herstellung eines Arzneimittels zur Durchführung von Gentherapie bei einem Säugerindividuum, wobei das Arzneimittel direkt in den Lungenkreislauf des Säugerindividuums zu verabreichen ist.

8. Verwendung nach Anspruch 7, wobei die genetisch veränderten Zellen Hautfibroblastenzellen sind.

9. Verwendung nach Anspruch 7 oder 8, wobei das exprimierbare Transgen ein Gen ist, das einen angiogenen Faktor codiert.

10. Verwendung nach einem der Ansprüche 7 bis 9, wobei das exprimierbare Gen ein Gen ist, das einen angiogenen Faktor codiert, ausgewählt aus Stickoxidsynthase, einem vaskulären endothelialen Wachstumsfaktor, Fibroblasten-Wachstumsfaktor, Angiopoietin-1, Transformierenden Wachstumsfaktor-$\beta$ und hepatischen Wachstumsfaktor.

11. Verwendung nach Anspruch 10, wobei der angiogene Faktor vaskulärer endothelialer Wachstumfaktor, VEGF, ist.

12. Verwendung von ex vivo genetisch veränderten Hautfibroblastenzellen, die ein exprimierbares Transgen enthalten, das einen angiogenen Faktor codiert, für die Herstellung eines Arzneimittels, das gentechnisch veränderte Zellen umfasst, zur Linderung der Symptome von Lungenhochdruck bei einem daran leidenden Säugerindividuum, wobei das Arzneimittel direkt in den Lungenkreislauf des Individuums zu verabreichen ist.

13. Verwendung von lebensfähigen, transfizierten Säugerfibroblasten, die zumindest ein exprimierbares Cystisches

**EP 1 839 667 B1**

Fibrose-Transgen enthalten, für die Herstellung eines Arzneimittels zur Linderung einer Lungenkrankheit oder Symptomen davon bei einem Individuum, wobei das Arzneimittel direkt in den Lungenkreislauf eines Säugerindividuums zu verabreichen ist.

14. Verwendung nach Anspruch 13, wobei die Säugerzellen von dem späteren Empfänger der transfizierten Zellen stammen.

15. Verwendung von *ex vivo* genetisch veränderten Säugerfibroblasten, die zumindest ein exprimierbares Cystisches Fibrose-Transgen enthalten, das mindestens ein Genprodukt nach Verabreichung in der Lunge exprimieren kann, für die Herstellung eines Arzneimittels zur Durchführung von Gentherapie bei einem Säugerindividuum, wobei das Arzneimittel direkt in den Lungenkreislauf des Säugerindividuums zu verabreichen ist.


**Revendications**

1. Utilisation de cellules de mammifères transfectées viables sélectionnées à partir de fibroblastes et de cellules endothéliales, lesdites cellules de mammifères transfectées contenant au moins un transgène exprimable codant pour un facteur angiogénique ou vasoactif pour la préparation d'une composition pharmaceutique destinée à soulager un trouble pulmonaire chez un patient ou ses symptômes, dans laquelle ladite composition pharmaceutique doit être administrée à la circulation pulmonaire d'un patient mammifère.

2. Utilisation selon la revendication 1, dans laquelle les cellules de mammifères sont des fibroblastes dermiques.

3. Utilisation selon la revendication 1 ou 2, dans laquelle le facteur angiogénique est l'oxyde nitrique synthase, un facteur de croissance de l'endothélium vasculaire, un facteur de croissance des fibroblastes, une angiopoïétine 1, un facteur de croissance transformant bêta, un facteur de croissance hépatique (facteur de dispersion) ou un facteur inductible par hypoxie (HIF).

4. Utilisation selon l'une quelconque des revendications 1 à 3, dans laquelle le facteur angiogénique est un facteur de croissance de l'endothélium vasculaire, le VEGF.

5. Utilisation selon l'une quelconque des revendications précédentes, dans laquelle les cellules de mammifères ont pour origine le receveur possible des cellules transfectées.

6. Utilisation selon l'une quelconque des revendications précédentes, dans laquelle le trouble pulmonaire est l'hypertension pulmonaire.

7. Utilisation de cellules de mammifères modifiées génétiquement *ex vivo* sélectionnées à partir de fibroblastes dermiques et de cellules endothéliales, contenant au moins un transgène exprimable qui est capable d'exprimer au moins un produit génétique dans la circulation pulmonaire après l'administration dans celle-ci, ledit transgène étant un transgène codant pour un facteur angiogénique, l'oxyde nitrique synthase, une lipoprotéine, une substance vasoactive, un antioxydant, un piégeur des radicaux libres, un récepteur de cytokine soluble, une molécule d'adhérence soluble, un récepteur soluble pour un virus ou une cytokine ; ou le gène de la fibrose cystique pour la préparation d'une composition pharmaceutique pour effectuer une thérapie génique chez un patient mammifère, dans laquelle ladite composition pharmaceutique doit être administrée à la circulation pulmonaire du patient mammifère.

8. Utilisation selon la revendication 7, dans laquelle les cellules génétiquement modifiées sont des cellules de fibroblastes dermiques.

9. Utilisation selon la revendication 7 ou 8, dans laquelle le transgène exprimable est un gène codant pour un facteur angiogénique.

10. Utilisation selon l'une quelconque des revendications 7 à 9, dans laquelle le gène exprimable est un gène codant pour un facteur angiogénique sélectionné à partir de l'oxyde nitrique synthase, d'un facteur de croissance endothélial vasculaire, d'un facteur de croissance de fibroblastes, d'une angiopoïétine 1, d'un facteur de croissance bêta transformant et d'un facteur de croissance hépatique.

**11.** Utilisation selon la revendication 10, dans laquelle le facteur angiogénique est un facteur de croissance de l'endothélium vasculaire, le VEGF.

**12.** Utilisation des cellules de fibroblastes dermiques modifiées génétiquement *ex vivo* contenant un transgène exprimable codant pour un facteur angiogénique pour la préparation d'une composition pharmaceutique comprenant des cellules modifiées génétiquement, pour soulager les symptômes de l'hypertension pulmonaire chez un patient mammifère souffrant de celle-ci, dans laquelle ladite composition pharmaceutique doit être administrée à la circulation pulmonaire du patient mammifère.

**13.** Utilisation de fibroblastes de mammifères transfectés viables contenant au moins un transgène de la fibrose cystique exprimable pour la préparation d'une composition pharmaceutique pour soulager un trouble pulmonaire chez un patient ou ses symptômes, dans laquelle ladite composition pharmaceutique doit être administrée à la circulation pulmonaire d'un patient mammifère.

**14.** Utilisation selon la revendication 13, dans laquelle les cellules de mammifères ont pour origine le receveur possible des cellules transfectées.

**15.** Utilisation de fibroblastes de mammifères modifiés génétiquement *ex vivo* contenant au moins un transgène de la fibrose cystique exprimable qui est capable d'exprimer au moins un produit de gène dans le poumon après y avoir été administré, pour la préparation d'une composition pharmaceutique pour effectuer une thérapie génique chez un patient mammifère, dans laquelle ladite composition pharmaceutique doit être administrée à la circulation pulmonaire du patient mammifère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 07009031 A

**Non-patent literature cited in the description**

- **SCHACHTNER, S.K. ; J.J. ROME ; R.F. HOYT, JR. ; K.D. NEWMAN ; R. VIRMAN ; D.A. DICHEK.** In vivo adenovirus-mediated gene transfer via he pulmonary artery of rats. *Circ. Res.,* 1995, vol. 76, 701-709
- **RODMAN, D.M. ; H. SAN ; R. SIMARI ; D. STEPHAN ; F. TANNER ; Z. YANG ; G.J. NABEL ; E.G. NABEL.** In vivo gene delivery to the pulmonary circulation in rats: transgene distribution and vascular inflammatory response. *Am. J. Respir. Cell Mol. Biol.,* 1997, vol. 16, 640-649

- **RODMAN, D.M. ; H. SAN ; R. SIMARI ; D. STEPHAN ; F. TANNER ; Z. YANG ; G.J. NABEL ; E.G. NABEL.** In vivo gene delivery to the pulmonary circulation in rats: transgene distribution and vascular inflammatory response. *Am. J. Respir. Cell Mol. Biol,* 1997, vol. 16, 640-649
- **NABEL, E.G. ; Z. YANG ; D. MULLER ; A.E. CHANG ; X. GAO ; L. HUANG ; K.J. CHO ; G.J. NABEL.** Safety and toxicity of catheter gene delivery to the pulmonary vasculature in a patient with metastatic melanoma. *Hum. Gene Ther,* 1994, vol. 5, 1089-1094